Europäisches Patentamt

(19) European Patent Office

Office européen des brevets

(11) Veröffentlichungsnummer: **0 216 314**
**B1**

(12) **EUROPÄISCHE PATENTSCHRIFT**

(45) Veröffentlichungstag der Patentschrifft:
21.03.90

(21) Anmeldenummer: 86112906.2

(22) Anmeldetag: 18.09.86

(51) Int. Cl. ⁵: **C 07 C 31/20, C 07 C 29/16**

(54) Verfahren zur Herstellung von Nonadecandiolen.

(30) Priorität: 26.09.85 DE 3534317

(43) Veröffentlichungstag der Anmeldung:
01.04.87 Patentblatt 87/14

(45) Bekanntmachung des Hinweises auf die Patenterteilung:
21.03.90 Patentblatt 90/12

㊻ Bennante Vertragsstaaten:
AT BE DE FR GB IT

(56) Entgegenhaltungen:
DE-A-2 410 156
DE-A-2 627 354
DE-A-2 914 189
DE-A-3 017 682
DE-A-3 235 030
US-A-4 216 343
US-A-4 329 521
US-A-4 451 680

(73) Patentinhaber: HOECHST AKTIENGESELLSCHAFT
Postfach 80 03 20
D-6230 Frankfurt am Main 80 (DE)

(72) Erfinder: Bahrmann, Helmut, Dr. Dipl.Chem.
Rohstrasse 48
D-4236 Hamminkeln 3 (DE)
Erfinder: Cornils, Boy, Dr. Dipl.-Chem.
Kirschgartenstrasse 6
D-6238 Hofheim (DE)
Erfinder: Konkol, Werner, Dr. Dipl.-Chem.
Lützowstrasse 40 a
D-4200 Oberhausen 11 (DE)
Erfinder: Weber, Jürgen, Dr. Dipl.-Chem.
Bunsenstrasse 17
D-4200 Oberhausen 11 (DE)
Erfinder: Bexten, Ludger, Dr. Dipl.-Chem.
Im Freihof 9
D-4224 Hünxe (DE)
Erfinder: Bach, Hanswilhelm, Dr. Dipl.-Chem.
Alleestrasse 56
D-4100 Duisburg 11 (DE)

LIBERGRAF, STOCKHOLM 1990

**Beschreibung**

Die vorliegende Erfindung betrifft ein Verfahren zur Herstellung von Nonadecandiolen durch Hydroformylierung von Oleylalkohol in Gegenwart von Rhodium-Komplex-Katalysatoren und Hydrierung des Hydroformylierungsproduktes nach Abtrennung des Katalysators.

Es ist bekannt, durch Umsetzung von Olefinen mit Kohlenmonoxid und Wasserstoff Aldehyde und Alkohole herzustellen. Die Reaktion wird durch Hydridometallcarbonyle, vorzugsweise solcher der Metalle der 8. Gruppe des Periodensystems, katalysiert. Neben Kobalt, das als Katalysatormetall in großem Umfang technische Anwendung findet, gewinnt in letzter Zeit Rhodium zunehmende Bedeutung. Im Gegensatz zu Kobalt gestattet Rhodium die Reaktion bei niedrigem Druck durchzuführen; darüber hinaus werden in Anwesenheit von überschüssigem Phosphin als Komplexierungsmittel vorzugsweise geradkettige n-Aldehyde und nur in untergeordnetem Maße iso-Aldehyde gebildet. Schließlich ist auch die Hydrierung der Olefine zu gesättigten Kohlenwasserstoffen bei Anwendung von Rhodium-Katalysatoren deutlich niedriger als bei Anwendung von Kobalt-Katalysatoren.

Bei den in der Technik eingeführten Verfahren wird der Rhodium-Katalysator in Form von modifizierten Hydridorhodiumcarbonylen eingesetzt, die zusätzliche und gegebenenfalls überschüssige Liganden enthalten. Besonders bewährt haben sich als Liganden tertiäre Phosphine oder Phosphite. Ihre Anwendung ermöglicht, den Reaktionsdruck auf Werte unter 30 MPa zu senken.

Probleme wirft bei diesem Verfahren jedoch die Abtrennung der Reaktionsprodukte und die Wiedergewinnung der im Reaktionsprodukt homogen gelösten Katalysatoren auf. Im allgemeinen destilliert man hierzu das Umsetzungsprodukt aus dem Reaktionsgemisch ab. In der Praxis kann dieser Weg wegen der thermischen Empfindlichkeit der gebildeten Aldehyde und Alkohole aber nur bei der Hydroformylierung niedriger Olefine, d. h. Olefine mit bis zu etwa 5 Kohlenstoffatomen im Molekül, beschritten werden.

Bei der Hydroformylierung langkettiger Olefine werden Produkte mit hohem Siedepunkt gebildet, die sich destillativ vom homogen gelösten Rhodium-Komplexkatalysator nicht abtrennen lassen. Die thermische Belastung des Destillationsgutes führt durch Dickölbildung zu erheblichen Verlusten an Wertprodukten und an Katalysator durch Zersetzung der Rhodiumkomplexverbindungen.

Die Abtrennung des Katalysators auf thermischem Wege wird durch Anwendung in Wasser löslicher Katalysatorsysteme vermieden. Derartige Katalysatoren sind z. B. in der DE-PS-2 627 354 beschrieben. Die Löslichkeit der Rhodiumkomplexverbindungen wird hierbei durch Verwendung von sulfonierten Triarylphosphinen als Komplexbestandteil erreicht. Die Abtrennung des Katalysators vom Reaktionsprodukt nach Beendigung der Hydroformylierungsreaktion erfolgt bei dieser Verfahrensvariante einfach durch Trennung von wässriger und organische Phase, d. h. ohne Destillation und damit ohne zusätzliche thermische Verfahrensschritte.

Ein weiteres Merkmal dieser Arbeitsweise ist, daß mit hoher Selektivität aus endständigen Olefinen n-Aldehyde und nur in ganz untergeordnetem Maße iso-Aldehyde gebildet werden. Neben sulfonierten Triarylphosphinen werden als Komplexbestandteile wasserlöslicher Rhodiumkomplexverbindungen auch carboxylierte Triarylphosphine eingesetzt.

Der Einsatz wasserlöslicher Katalysatoren hat sich bei der Hydroformylierung niederer Olefine, insbesondere Ethylen und Propylen ausgezeichnet bewährt. Setzt man höhere Olefine wie Hexen, Octen oder Decen ein, gehen der Umsatz und/oder die Selektivität der Reaktion zu n-Verbindungen merklich zurück. Die Wirtschaftlichkeit der Umsetzung in technischem Maßstab ist dann häufig nicht mehr gegeben.

Ein Verfahren zur Hydroformylierung von Oleylalkohol ist in der US-PS-4 216 343 beschrieben. Als Katalysator wird Rhodium auf einem Träger zusammen mit überschüssigem Triphenylphosphit eingesetzt. Die Reaktion des Alkohols mit Synthesegas erfolgt bei 130°C und 70 bis 75 bar. Statt Triphenylphosphit kann auch Triphenylphosphin verwendet werden.

Das Reaktionsprodukt Formyloctadecanol wird anschließend ohne vorherige Katalysatorabtrennung mit methanolischer Formaldehydlösung in Gegenwart von Alkali zu den geminalen Bis-(hydroxymethyl)-octadecanolen umgesetzt.

Alternativ kann zunächst das Rhodium enthaltende Formyloctadecanol mit Formaldehyd zu dem entsprechenden Hydroxymethylformyloctadecanol umgesetzt werden. Darauf hydriert man entweder mit Wasserstoff in Gegenwart eines Kupferchromit-Katalysators oder mit $LiAlH_4$ zu dem entsprechenden 9,9(10,10)-Bis(hydroxymethyl)octadecanol.

Beide Wege zur Hydrierung des Formyloctadecanols befriedigen nicht. Die Anwendung alkalischer Formaldehydlösung führt nicht nur zu einer Vergrößerung des Reaktionsvolumens, sondern hat auch das Auftreten von Konkurrenzreaktionen und damit die Bildung von Nebenprodukten zur Folge. $LiAlH_4$ ist ein sehr teures Hydrierreagenz und beeinträchtigt daher erheblich die Wirtschaftlichkeit des Verfahrens.

Die DE-OS-2 914 189 betrifft die Herstellung von Nonadecandiolen durch Hydroformylierung von Oleylalkohol und nachfolgende Hydrierung in einer Stufe. Als Katalysator für beide Reaktionsschritte dient Rhodium in Gegenwart eines sehr hohen Überschusses an tertiärem Amin. Die Umsetzung erfolgt bei einem Gesamtdruck von 20 MPa (7,5 MPa CO, 12,5 MPa $H_2$) und einer von 130°C auf 180°C ansteigenden Temperatur. Die Diol-Ausbeute liegt bei 71 bis 76 % der Theorie. Der Rhodium enthaltende Katalysator verbleibt im Reaktionsgemisch. Auf seine Abtrennung aus dem Reaktionsgemisch und seine Wiederaufarbeitung wird in der zitierten Druckschrift nicht eingegangen.

Die hohe Reaktionstemperatur führt zu einer Zerstörung des katalytisch wirkenden Rhodiumkomplexes. Der Katalysator wird daher desaktiviert und kann nicht wieder in die Reaktion zurückgeführt werden. Überdies besteht die Gefahr, daß sich metallisches Rhodium im Reaktor abscheidet und nicht oder nur mit großem Aufwand wiedergewonnen werden kann.

Es bestand daher die Aufgabe, ein Verfahren zu entwickeln, das es erlaubt, Oleylalkohol in hoher Ausbeute zu hydroformylieren, aus dem Hydroformylierungsprodukt den Katalysator unter schonenden Bedingungen möglichst vollständig abzutrennen und den als Zwischenprodukt gebildeten Formylalkohol auf konventionellem Wege zum Diol zu hydrieren.

Diese Aufgabe wird gelöst durch ein Verfahren zur Herstellung von Nonadecandiolen durch Hydroformylierung von Oleylalkohol bei 100 bis 170°C und 10 bis 45 MPa in homogener Phase und in Gegenwart eines Rhodium sowie aromatische Phosphine im molaren Überschuß, bezogen auf Rhodium, enthaltenden Katalysatorsystems, Abtrennung des Katalysators und Hydrierung des Hydroformylierungsproduktes, dadurch gekennzeichnet, daß man als aromatische Phosphine in organischen Medien lösliche in Wasser unlösliche Salze sulfonierter oder carboxylierter Triarylphosphine einsetzt, das Hydroformylierungsprodukt mit einer verdünnten wässrigen Lösung einer Base behandelt, die wässrige, Phosphin und Rhodium enthaltende Phase abtrennt und das Hydroformylierungsprodukt unter erhöhter Temperatur mit Wasserstoff in Gegenwart eines Hydrierkatalysators behandelt.

Die erfindungsgemäße Arbeitsweise vereinigt die Vorteile bekannter Hydroformylierungsverfahren, ohne deren Nachteile zu besitzen. Einerseits gestattet es die Hydroformylierung von Oleylalkohol in homogener Phase und stellt damit einen hohen Umsatz sicher. Andererseits ermöglicht es eine schonende und weitgehende Abtrennung des Katalysators vor der Hydrierung des Formyloctadecanols zu dem entsprechenden Diolgemisch.

Das Katalysatorsystems besteht aus Salzen sulfonierter oder carboxylierter Triarylphosphine, die zwar in organischen Medien löslich, aber in Wasser unlöslich sind, und dem über das Phosphoratom komplex gebundenen Rhodium. Die Kationen der Salze weisen die Gruppierung $[NR_2H_2]^+$ und/oder $[NR_3H]^+$ auf, wobei R für Alkylreste mit 4 bis 12 Kohlenstoffatomen, für Aryl- oder Cycloalkylreste mit 6 bis 12 Kohlenstoffatomen steht.

Die Salze der sulfonierten oder carboxylierten Triarylphosphine werden der Hydroformylierung in gelöster Form zugesetzt. Es ist jedoch auch möglich, sie in fester Form dem in die Hydroformylierung eingesetzten Oleylalkohol beizufügen und, falls erforderlich, ihre Löslichkeit durch Zugabe von Lösungsmitteln zu verbessern. Geeignete Lösungsmittel sind Benzol, Toluol, Xylol und Cyclohexan.

Die Hydroformylierung des Oleylalkohols erfolgt bei 100 bis 170°C und 10 bis 45 MPa (100 bis 450 bar) in Gegenwart von 30 bis 150 ppm Rhodium, vorzugsweise 50 bis 100 ppm Rhodium, bezogen auf eingesetzten Oleylalkohol. Die Salze der sulfonierten oder carboxylierten Triarylphosphine werden im Verhältnis 5 : 1 bis 200 : 1, vorzugsweise 40 : 1 bis 100 : 1 (Mol Triarylphosphinsalz je g-Atom Rhodium) eingesetzt. Die Synthesegaszusammensetzung kann zwischen einem Verhältnis CO : $H_2$ von 10 : 1 bis 1 : 10 schwanken; sie liegt üblicherweise bei 1 : 1.

Als besonders brauchbare sulfonierte Triarylphosphine haben sich mono-, di- oder trisulfoniertes Triphenylphosphin, nämlich $(C_6H_5)_2PC_6H_4SO_3H$, $C_6H_5P(C_6H_4SO_3H)_2$ bzw. $P(C_6H_4SO_3H)_3$ erwiesen.

Aber auch Mischungen mono-, di- und trisulfonierter Triphenylphosphine sind als Katalysatorkomponente geeignet.

Nach der Hydroformylierung wird das Reaktionsgemisch mit einer verdünnten wässrigen Lösung einer wasserlöslichen Base behandelt.

Die zur Spaltung der in organischen Medien löslichen, in Wasser unlöslichen Salze sulfonierter oder carboxylierter Triarylphosphine benötigten wasserlöslichen Basen müssen hinreichend alkalisch sein, um den gewünschten pH-Wert bei der Extraktion des Hydroformylierungsgemisches mit der verdünnten, wässrigen Lösung der Base einzustellen. Diese Anforderung erfüllen die Alkali- und Erdalkalihydroxide, es können aber auch wässrige Tetraalkylammoniumhydroxid-Lösungen verwendet werden. Die Konzentration der wasserlöslichen Base beträgt 0,01 bis 10 Gew.-%, vorzugsweise 0,5 bis 5 Gew.-%, bezogen auf wässrige Lösung.

Durch Vermischen beider Phasen bei Temperaturen ≤ 70°C, vorzugsweise ≤ 40°C, werden aus den $[NR_2H_2]^+$- oder $[NR_3H]^+$-Salzen die entsprechenden sekundären oder tertiären Amine freigesetzt, gleichzeitig wird ein wasserlösliches Salz des sulfonierten oder carboxylierten Triarylphosphins gebildet. Dieses gelangt durch Extraktion in die wässrige Phase und wird zusammen mit dem komplex am Phosphor gebundenen Rhodium abgetrennt.

Während dieser Extraktion soll der pH-Wert des Gemisches beider Phasen ≥ 8, vorzugsweise ≥ 8,5 betragen. Im allgemeinen empfiehlt es sich, einen pH-Bereich von 8 bis 10, vorzugsweise 8,5 bis 9 einzuhalten.

Die Extraktion ist einfach auszuführen. Infolge der verringerten thermischen Belastung wird einerseits die Desaktivierung und thermische Zersetzung und damit die Schädigung des Katalysatorsystems herabgesetzt, andererseits wird die Bildung unerwünschter Nebenprodukte, die aus dem Hydroformylierungsprodukt entstehen, vermindert.

Die Rückgewinnungsquote ist bereits im ersten Extraktionsschritt ungewöhnlich hoch und liegt > 90 Gew.-%, bezogen auf eingesetztes Rhodium. Durch mehrfache Extraktion kann dieses Resultat weiter verbessert werden.

Organische und wässrige Phase trennen sich schnell und vollständig voneinander. Um die Entmischung der Phasen zu beschleunigen, zentrifugiert man gegebenenfalls und separiert anschließend die obere organische Phase von der unteren wässrigen Phase. Mit Erfolg werden auch Trennelemente mit Koalezierelementen eingesetzt, (wie z. B. Franken-Filter).

Nach Abtrennung der Rhodium und Triarylphosphin enthaltenden Wasserphase wird das Hydroformylierungsproduktfalls erforderlich - mehrfach mit kaltem Wasser gewaschen, um noch vorhandene basische Anteile zu beseitigen. Diese Reinigung muß sorgfältig erfolgen, um basisch katalysierte Nebenreaktionen, z. B. Aldolisierungen während der Hydrierung zu vermeiden.

Das vom Hydroformylierungskatalysator befreite rohe Formyloctanol wird bei erhöhter Temperatur in Gegen-

EP 0 216 314 B1

wart eines Katalysators hydriert. Die Hydrierung kann in der Gasphase, in Suspension oder in der Flüssigphase durchgeführt werden.

Die Katalysatoren enthalten Kupfer, Kupferchromit, Kobalt oder Nickel, gegebenenfalls sind als Promotoren daneben noch Erdalkali, Zink, Aluminium und/oder Chrom enthaltende Verbindungen vorhanden. Sie können reine Metallkatalysatoren darstellen oder auf Trägermaterialien aufgebracht sein. Besonders bewährt haben sich Trägerkatalysatoren mit 25 bis 65 Gew.-% Metall, vorzugsweise 40 bis 60 Gew.-% Metall, bezogen auf die gesamte Katalysatormasse. Als Trägermaterialien finden Bimsstein, Kieselerde, Tonerde, Aluminiumoxid und $SiO_2$ in seinen verschiedenen Erscheinungsformen Anwendung.

Als besonders geeignet hat sich ein Nickel-Trägerkatalysator mit einem Gehalt von 55 Gew.-% Nickel und etwa 30 Gew.-% $SiO_2$, bezogen jeweils auf die gesamte Katalysatormasse, erwiesen.

Brauchbare Lösungsmittel für die Hydrierung sind Cyclohexan, Methylcyclohexan, Methanol, Ethanol, 2-Etylhexanol und andere höhere Alkohole. Es kann jedoch auch auf ein Lösungsmittel verzichtet werden.

Die Temperaturen liegen je nach Art des verwendeten Katalysators und in Abhängigkeit von der Verweilzeit des zu hydrierenden Produktes in einem Bereich von 80 bis 220°C. Bei Gasphasenfahrweise liegt der Druck bei 0,01 bis 15 MPa. Wird das Rohprodukt in der Flüssigphase hydriert, so liegen die Drücke entsprechend höher und betragen 5 bis 35 MPa, vorzugsweise 10 bis 25 MPa.

Die Reaktionszeit beträgt in Abhängigkeit von den gewählten Reaktionsbedingungen 1 bis 10 Stunden vorzugsweise 2 bis 6 Stunden.

Ein weiterer Vorteil des erfindungsgemäßen Verfahrens besteht darin, daß aus der wässrigen, Rhodium enthaltenden Phase ohne großen Aufwand der aktive Hydroformylierungskatalysator wieder zurückgewonnen werden kann. Man säuert den Extrakt z. B. mit Mineralsäuren bis zu einem pH-Wert von etwa 1 an und extrahiert die wässrige Phase mit einem in einem organischen Lösungsmittel (z. B. Benzol oder Toluol) gelösten sekundären bzw. tertiären Amin entsprechend der oben dargelegten Zusammensetzung. Dabei bildet sich das ursprünglich eingesetzte Aminsalz des sulfonierten oder carboxylierten Triarylphosphins zurück. Da dieses in Wasser nicht löslich ist, geht es zusammen mit dem komplex gebundenen Rhodium in die organische Phase über. Der so gewonnenen Reextrakt kann direkt als Hydroformylierungskatalysator Verwendung finden. Falls erforderlich, ergänzt man noch Rhodium und/oder den Phosphor (III) - Liganden.

In den folgenden Beispielen wird die Erfindung näher beschrieben.

Die nachfolgend verwendeten Abkürzungen bedeuten:

TPPDS       :    Triphenylphosphindisulfonsäure-salz
TPPTS       :    Triphenylphosphintrisulfonsäure-salz
TPPODS      :    Triphenylphosphinoxiddisulfonsäure-salz
TPPOTS      :    Triphenylphosphinoxidtrisulfonsäure-salz
TPPSTS      :    Triphenylphosphinsulfidtrisulfonsäure-salz

**Versuch 1**

Herstellung des Triisooctylammonium-Salzes von TPPTS (Phosphinmischungen I und II).

Die Sulfonierung von Triphenylphosphin mittels Oleum und die sich hieran anschließende Weiterverarbeitung ist in der DE-3 235 030 Al beschrieben.

In Anlehnung an diese Arbeitsweise wird Triphenylphosphin bei Raumtemperatur mit Oleum umgesetzt und das entstandene Gemisch durch Zusatz von kaltem Wasser hydrolisiert (s. Tab. 1, Spalte 1). Anschließend fügt man eine Lösung von Triisooctylamin in Toluol zu und rührt etwa 30 Minuten. Nach Beendigung des Rührens wird die untere Schwefelsäure enthaltende Wasserphase abgetrennt. Durch Zusatz von 3 %-iger wässriger Natronlauge wird ein pH-Wert von 4,6 eingestellt, die Wasserphase abgetrennt und verworfen. Anschließend wird die toluolische Lösung noch zweimal mit Wasser gewaschen. Zur Vermeidung unerwünschter Oxidation des Phosphins wird unter striktem Sauerstoffausschluß gearbeitet. Auf diese Weise werden zwei Phosphin-Mischungen I und II hergestellt, deren analytische Daten in der Tabelle 1 zusammengestellt sind.

**Tabelle 1**

(Werte mittels HPLC-Analyse als Natriumsalze bestimmt)

| | Sulfonierungs-[1] rohprodukt | Phosphin-[2] Mischung I | Phosphin-[2] Mischung II |
|---|---|---|---|
| TPPDS | 0,441 Gew. | 1,714 Gew.-% | 0,74 Gew.-% |
| TPPTS | 2,58 " | 8,087 " | 5,01 " |
| TPPODS | 0,048 " | 0,205 " | 0,42 " |
| TPPOTS | 0,578 " | 0,809 " | 1,20 " |
| P(III)[3] | 0,055 mol | 0,165 mol/kg | 0,105 mol/kg |

[1] Wässrige, schwefelsaure Lösung  [2] Aminsalzlösung in Toluol  [3] jodometrisch bestimmt

4

**Versuch 2**

Herstellung des Tri-(di-2-ethylhexylammonium)-Salzes von TPPTS (Phosphinmischung III)

5130 g eines bereits mit kaltem Wasser hydrolysierten Triphenylphosphin-Sulfonierungsgemisches (Zusammensetzung siehe Tabelle 1: Sulfonierungsrohprodukt) werden unter $N_2$-Schutz bei 20°C mit 212 g (0,876 mol) Di-(2-ethylhexyl)-amin gelöst in 848 g Toluol, versetzt. Man rührt 2 Stunden und läßt danach über Nacht stehen. Es bilden sich drei Phasen, die obere Phase (438 g) besteht aus Toluol, die mittlere Phase (742 g) enthält das Amin-Salz des TPPTS. Sein P (III)-Gehalt beträgt 0,342 mol/kg (jodometrisch bestimmt).

Die untere Phase (5113 g) enthält Wasser und Schwefelsäure.

**Versuch 3**

Herstellung des Tri-(di-n-hexylammonium)-Salzes von TPPTS (Phosphinmischung IV)

1273 g des in Versuch 1 verwendeten Hydrolysegemisches werden unter $N_2$-Schutz bei 20°C mit 38,9 g (210 mmol) Dihexylamin gelöst in 156 g Toluol, versetzt. Man rührt 2 Stunden und läßt über Nacht stehen. Es bilden sich drei Phasen. Die obere Schicht (101 g) besteht aus Toluol, die mittlere Schicht (120 g) enthält das Dihexyl-amin-Salz von Di- bzw. Trisulfophenylphosphin gelöst in Toluol, während die untere Phase (1229 g) nur wässrige Schwefelsäure enthält und abgetrennt wird.

Auf eine Behandlung der toluolischen Lösung des Amin-Salzes mit wässriger Natronlauge (Einstellung des pH-Wertes) wird verzichtet. Der pH-Wert der Lösung liegt bei 1. Sein P (III)-Gehalt beträgt 0,121 mol/kg.

**Beispiel 1**

**Herstellung von Nonadecandiol aus Oleylalkohol**

**a) Hydroformylierung von Oleylalkohol**

In einem 2 l-Autoklaven, der einen Rotationsrührer besitzt, werden 519 g HD-Ocenol 80/85 (Handelsprodukt der Fa. Henkel; 63 Gew.-% Oleylalkohol), 80 g Phosphin-Mischung I und 25 mg Rhodium in Form des Rh (III)-2-Ethylhexanoats vorgelegt. Es wird unter $N_2$-Schutz gearbeitet. Der Druck (27 MPa) wird mit Synthesegas ($CO/H_2$ = 1 : 1) eingestellt. Die Reaktion läuft über 6 Std. bei 130°C ab, verbrauchtes Synthesegas wird durch Nachpressen ergänzt. Anschließend wird der Autoklav abgekühlt, entspannt und das Reaktionsprodukt gaschromatographisch untersucht. 0,5 Gew.-% Oleylalkohol bleiben nicht umgesetzt zurück.

**b) Abtrennung des Rhodiumkatalysators**

247 g des unter a) anfallenden Hydroformylierungsproduktes werden in einen Dreihalskolben überführt und intensiv gerührt. Anschließend wird 8 %-ige wässrige Natronlauge (9 ml) zugegeben und ein pH-Wert von 8,9 eingestellt. Um die Entmischung der beiden Phasen zu beschleunigen, wird das Gemisch mit einer Laborzentrifuge (4500 Umdrehungen/Min. 5 Min. lang zentrifugiert. Es entstehen zwei Phasen, deren untere, wässrige Phase einen unlöslichen Bodensatz aufweist.

In der oberen organischen Phase (235,3 g) befinden sich nur noch 3,8 Gew.-%, in der unteren Phase (10,34 g) dagegen 94,8 Gew.-% und in dem unlöslichen Rückstand (0,55 g) 1,4 Gew.-% des ursprünglich im Hydroformylierungsgemisch enthaltenen Rhodiums.

**c) Hydrierung des Hydroformylierungsproduktes**

Das gemäß b) erhaltene, von Rhodium und P (III)-Ligand befreite Hydroformylierungsprodukt wird an einem Nickel-Trägerkatalysator, der 55 Gew.-% Ni und etwa 30 Gew.-% $SiO_2$ bezogen auf gesamte Katalysatormasse besitzt, hydriert. Es werden 5 Gew.-% Nickelkatalysator, bezogen auf eingesetztes organisches Material, verwendet, der Druck beträgt 10 MPa, die Temperatur 150°C und die Reaktionszeit 2 Stunden. Der verwendete Autoklav besitzt eine Rührvorrichtung. Sein Volumen beträgt 1 l.

Die gaschromatografischen Analysen der Einsatz-, Zwischen- und Endprodukte sind in der nachfolgenden Tabelle zusammengestellt.

**Tabelle 2**

| Einsatzprodukt: HD-Ocenol 80/85 | Gew.-%*) | Hydroformylierungsprodukt | Gew.-% | Hydrierprodukt | Gew.-% |
|---|---|---|---|---|---|
| Vorlauf | 1,66 | $C_{12} - C_{15}$-Alkohole | 4,2 | | |
| $C_{10} - C_{15}$-Alkohole | 4,31 | | | n-$C_{14}$-Alkohol | 2,4 |
| n-$C_{16}$-Alkohol | 9,15 | n-$C_{16}$-Alkohol | 10,54 | n-$C_{16}$-Alkohol | 12,7 |
| ungesättigte $C_{16}$-Alkohole | 5,13 | $C_{17}$- + isomere $C_{18}$-Alkohole | 2,12 | Zwischenlauf: | 2,6 |
| Zwischenlauf: | 1,74 | Zwischenlauf: | 5,9 | n-$C_{17}$-Alkohol + Triisooctylamin | 6,1 |
| n-$C_{18}$-Alkohol | 5,15 | n-$C_{18}$-Alkohol | 7,0 | n-$C_{18}$-Alkohol + Triisooctylamin | 10,7 |
| n-$C_{18}$-Alkohol einfach ungesättigt | 62,69 | Formyl-n-$C_{18}$-Alkohol | 55,4 | $C_{19}$-Diol | 62,7 |
| n-$C_{18}$-Alkohole, mehrfach ungesättigt | 8,55 | andere Formylalkohole | 4,81 | andere Diole + Triole | 2,8 |
| n-$C_{20}$-Alkohol | 1,55 | eluierbarer Nachlauf | 0,2 | | |
| $C_{22}$-Alkohol | 0,07 | Triisooctylamin | 9,8 | | |

\* alle Werte gaschromatografisch bestimmt

**d) Destillative Aufarbeitung des hydrierten Produktgemisches**

112 g des gemäß c) erhaltenen hydrierten Produktgemisches werden durch Filtration von Katalysatorresten befreit und in einer einfachen Destillationsapparatur (Vigreux-Kolonne von 10 cm Länge) bei einem Druck von 0,39 kPa destilliert. Der Vorlauf geht bei 73 bis 203°C über. Hierin sind Toluol, niedere Alkohole sowie Triisooctylamin enthalten.

Der Hauptlauf (63 g), der bei 203 bis 220°C übergeht, weist einen Gehalt an Nonadecandiol von 84,4 Gew.-% bezogen auf Hauptlauf auf.

Der Anteil an Höhersiedern (Dicköl) beträgt 10,7 Gew.-% (bezogen auf eingesetztes Produkt).

**(Beispiel 2)**

**Herstellung von Nonadecandiol aus Oleylalkohol**

**a) Hydroformylierung von Oleylalkohol**

In einem 1 l-Autoklaven, der einen Rotationsrührer besitzt, werden 273 g HD-Ocenol 80/85 (Handelsprodukt der Firma Henkel, 63 Gew.-% Oleylalkohol) 126,5 g Phosphin-Mischung II) entsprechend 13,3 mmol P(III) und 27 mg Rhodium in Form des Rh (III) 2-Ethylhexanoates vorgelegt und wie in Beispiel 1 beschrieben hydroformyliert.

Nach 6 Stunden hat sich der Oleylalkohol vollständig umgesetzt.

**b) Abtrennung des Rhodiumkatalysators (Vergleichsversuch)**

Das Hydroformylierungsprodukt wird mit wässriger Natronlauge wie in Beispiel 1 b) angegeben behandelt, aber in Abweichung zu Beispiel 1 b) nur ein pH-Wert von 7 eingestellt. Die Folge ist eine wesentlich verringerte Abtrennung des Rhodiums aus der organischen Phase. Die in der wässrigen Phase vorliegende Rhodiummenge beträgt nur 14,7 Gew.-% der eingesetzten Menge. Dies zeigt, daß für eine ausreichende Abtrennung des Rhodiums aus der organischen Phase ein pH-Wert oberhalb von 7 eingestellt werden muß.

Das von der wässrigen Phase befreite Hydroformylierungsprodukt wird wie in Beispiel 1c) beschrieben hydriert. Es weist einen Gehalt von 47,9 Gew.-% Nonadecandiol auf.

Analog Beispiel 1 d) werden 154 g des hydrierten Rohgemisches in eine einfache Destillationsapparatur (Vigreux-Kolonne von 10 cm Länge) gegeben und bei 0,26 kPa destilliert. Nach Abnahme des Vorlaufes fallen 18 g eines Zwischenlaufes, der einen Gehalt an Nonadecandiolen von 71,7 Gew.-% aufweist, an. Als Hauptlauf werden 51 g Produkt mit einem Gehalt an Nonadecandiolen von 85,0 Gew.-% isoliert.

Der Anteil der Höhersieder (Dicköl) liegt bei 9 Gew.-%, bezogen auf eingesetztes Produkt und damit nur geringfügig tiefer als in Beispiel 1 d).

**Beispiel 3**

**Herstellung von Nonadecandiol aus Oleylalkohol**

Die Hydroformylierung von Oleylalkohol wird entsprechend Beispiel 1 a) wiederholt. Anstelle von HD-Ocenol 80/85 wird HD-Ocenol 90/95 (Handelsprodukt der Fa. Henkel; 78,9 Gew.-% Oleylalkohol) verwendet. Als Ligand wird, wie in Beispiel 1 a) angegeben, die Phosphin-Mischung I verwendet.

Das Hydroformylierungsgemisch weist nach Abtrennung des Rhodiums (wie in 1 b) beschrieben, wobei 90 % des eingesetzten Rhodiums in die wässrige Phase übergehen) folgende Zusammensetzung auf: Toluol + Vorlaufkomponenten 2,5 Gew-%, Hexadecandol 4,1 Gew.-%, Octadecanol 5,2 Gew.-%, Triisooctylamin 12,3 Gew.-% und 68,6 Gew.-% Formyloctadecanol. Nach Hydrierung (wie in 1 c) beschrieben), erhält man ein Rohprodukt, das 75,3 Gew.-% Nonadecandiol beinhaltet.

**Beispiel 4**

**Herstellung von Nonadecandiol aus Oleylalkohol**

In einem 2 l-Autoklaven, der einen Rotationsrührer besitzt, werden 600 g HD-Ocenol 90/95 (Handelsprodukt der Firma Henkel, 78,9 Gew.-% Oleylalkohol), 187 g der Phosphinmischung III und 30 mg Rhodium in Form des Rh(III) 2-Ethylhexanoates sowie zusätzlich 9,3 g Di-2-ethylhexylamin gegeben.

Die Hydroformylierung erfolgt gemäß den Bedingungen von Beispiel 1 a). Das Hydroformylierungsgemisch enthält neben einigen Komponenten geringer Konzentration 7,2 Gew.-% Di-2-ethylhexylamin; 4,6 Gew.-% Hexadecanol; 3,9 Gew.-% Octadecanol; 10,3 Gew.-% Formylhexadecaol und 65,5 Gew.-% Formyloctadecanol. Die Abtrennung des Rhodiums erfolgt analog Beispiel 1 b), wobei 81, Gew.-% des eingesetzten Rhodiums in die wässrige Phase übergehen. Nach Hydrierung, die analog 1 c) durchgeführt wird, wird ein Rohprodukt erhalten, das 64,5 Gew.-% Nonadecandiol aufweist. Beispiel 4 belegt, daß die Hydroformylierung durch die Anwesenheit von freiem Amin nicht beeinträchtigt wird und auch nicht vermehrt Höhersieder gebildet werden.

**Beispiel 5**

**Herstellung von Nonadecandiol aus Oleylalkohol**

In einem 1 l-Autoklaven, der einen Rotationsrührer besitzt, werden 600 g HD-Ocenol 90/95 (Handelsprodukt der Firma Henkel, 78,9 Gew.-% Oleylalkohol), 99,2 g der Phosphin-Mischung IV, 30 mg Rhodium in Form des Rh(III) gegeben und es wird gemäß Beispiel 1 a) hydroformyliert.

Es findet jedoch kein Umsatz statt. Das aus dem Autoklaven entnommene Gemisch weist einen pH-Wert von 1,2 auf.

Das dem Autoklaven entnommene Gemisch wird unter Rühren mit wässriger Natronlauge (1 Gew.-%-ig) auf einen pH-Wert von 4,7 eingestellt, die wässrige Phase abgetrennt und die verbleibende organische Phase gemäß Beispiel 1 a) hydroformyliert. Es findet sofort Umsatz statt. Nach Beendigung der Reaktion besitzt das Hydroformylierungsrohprodukt einen Gehalt von 73 Gew.-% Formyloctanol. Nach Hydrierung analog Beispiel 1 c) erhält man ein Rohprodukt folgender Zusammensetzung:

3,5 Gew.-% Hexadecanol; 5,9 Gew.-% Octadecanol; 72,3 Gew.-% Nonadecandiol und 9,5 Gew.-% Höhersieder.

Über die Alkalisierung konnten 99 Gew.-% des Rhodiums in die wässrige Phase überführt werden.

Beispiel 5 belegt die Abhängigkeit der Hydroformylierung vom pH-Wert, der > 1,2; möglichst jedoch 4,5 bis 5,0 betragen sollte. Es empfiehlt sich daher bereits die als Ligand dienende Phosphin-Mischung auf diesen pH-Bereich einzustellen.

**Patentansprüche**

1. Verfahren zur Herstellung von Nonadecandiolen durch Hydroformylierung von Oleylalkohol bei 100 bis 170°C und 10 bis 45 MPa in homogener Phase und in Gegenwart eines Rhodium sowie aromatische Phosphine im molaren Überschuß, bezogen auf Rhodium enthaltenden Katalysatorsystems, Abtrennung des Katalysators und Hydrierung des Hydroformylierungsproduktes, dadurch gekennzeichnet, daß man als aromatische Phosphine in organischen Medium lösliche, in Wasser unlösliche Salze sulfonierter oder carboxylierter Triarylphosphine einsetzt, das Hydroformylierungsprodukt mit einer verdünnten wässrigen Lösung einer Base behandelt, die wässrige, Phosphin und Rhodium enthaltende Phase abtrennt und das Hydroformylierungsprodukt unter erhöhter Temperatur mit Wasserstoff in Gegenwart eines Hydrierkatalysators behandelt.

2. Verfahren nach Anspruch 1, dadurch gekennzeichnet, daß die in organischen Medien löslichen, in Wasser unlöslichen Salze sulfonierter oder carboxylierter Triarylphosphine als Kation $(NR_2H_2)^+$ und/oder $(NR_3H)^+$ enthalten, wobei R für Alkylreste mit 4 bis 12 Kohlenstoffatomen, für Aryl- oder Cycloalkylreste mit 6 bis 12 Kohlenstoffatomen steht.

3. Verfahren nach Anspruch 1 und 2, dadurch gekennzeichnet, daß die Hydroformylierung des Oleylalkohols in Gegenwart von 30 bis 150 ppm Rhodium, bezogen auf eingesetzten Oleylalkohol, erfolgt und die Salze der sulfonierten oder carboxylierten Triarylphosphine in einem molaren Verhältnis von 5 : 1 bis 200 : 1, bezogen auf Rhodium, eingesetzt werden.

4. Verfahren nach Anspruch 1 bis 3, dadurch gekennzeichnet, daß die Hydroformylierung des Oleylalkohols in Gegenwart von 50 bis 150 ppm Rhodium, bezogen auf eingesetzten Oleylalkohol, erfolgt und die Salze der sulfonierten oder carboxylierten Triarylphosphine in einem molaren Verhältnis von 40 : 1 bis 100 : 1, bezogen auf Rhodium, eingesetzt werden.

5. Verfahren nach Anspruch 1 bis 4, dadurch gekennzeichnet, daß die Abtrennung des Katalysatorsystems mittels der wässrigen Lösung der wasserlöslichen Base bei einer Temperatur von ≤ 70°C und einem pH-Wert von ≥ 8 durchgeführt wird.

6. Verfahren nach Anspruch 1 bis 5, dadurch gekennzeichnet, daß die Abtrennung des Katalysatorsystems mittels der wässrigen Lösung der wasserlöslichen Base bei einer Temperatur ≤ 40°C und einem pH-Wert von ≥ 8,5 durchgeführt wird.

7. Verfahren nach Anspruch 1 bis 6, dadurch gekennzeichnet, daß das von der wässrigen Phase abgetrennte Hydroformylierungsprodukt bei Temperaturen von 80 bis 220°C und unter Drücken von 0,01 bis 35 MPa an einem nickel-, kobalt- oder kupferhaltigen Katalysator, der gegebenenfalls Promotoren enthält, hydriert wird.

8. Verfahren nach Anspruch 1 bis 7, dadurch gekennzeichnet, daß das von der wässrigen Phase abgetrennte Hydroformylierungsprodukt bei Temperaturen von 80 bis 160°C und Drücken von 10 bis 25 MPa an einem nickelhaltigen Trägerkatalysator hydriert wird.

9. Verfahren nach Anspruch 1 bis 8, dadurch gekennzeichnet, daß das sulfonierte Triarylphosphin mono-, di- oder trisulfoniertes Triphenylphosphin ist.

10. Verfahren nach Anspruch 1 bis 9, dadurch gekennzeichnet, daß das sulfonierte Triarylphosphin aus Mischungen mono-, di- und trisulfonierter Triphenylphosphine besteht.

11. Verfahren nach Anspruch 1 bis 10, dadurch gekennzeichnet, daß als wasserlösliche Base Alkali-, Erdalkalihydroxide oder wässrige Tetraalkylammoniumhydroxid-Lösung verwendet werden.

12. Verfahren nach Anspruch 1 bis 11, dadurch gekennzeichnet, daß die Konzentration der in Wasser gelösten Base 0,01 bis 10 Gew.-%, vorzugsweise 0,5 bis 5 Gew.-%, bezogen auf wässrige Lösung, beträgt.

13. Verfahren nach Anspruch 1 bis 12, dadurch gekennzeichnet, daß die Abtrennung des Hydroformylierungsproduktes und der wässrigen Lösung durch Zentrifugieren oder durch Phasentrennaggregaten mit Koaleszierelementen herbeigeführt wird.

14. Verfahren nach Anspruch 1 bis 13, dadurch gekennzeichnet, daß man zur Rezirkulierung des Rhodiums die Phosphin und Rhodium enthaltende, vom Hydroformylierungsprodukt abgetrennte wässrige Phase ansäuert, mit einem in einem wasserunlöslichen organischen Lösungsmittel gelösten Amin der Formel $NHR_2$ bzw. $NR_3$, wobei R für Alkylreste mit 4 bis 12 Kohlenstoffatomen für Aryl- oder Cycloalkylreste mit 6 bis 12 Kohlenstoffatomen steht, behandelt und nach Abtrennung der wässrigen Phase die organische Phase wieder in die Hydroformylierung einsetzt.

15. Verfahren nach Anspruch 1 bis 14, dadurch gekennzeichnet, daß als Lösungsmittel für die aromatischen Phosphine Toluol, Xylol, arteigene Amine $NR_2H$ bzw. $NR_3$ oder das Olefin selbst dienen.

## Claims

1. A process for the preparation of nonadecane diols by the hydroformylation of oleyl alcohol at 100 to 170°C and 10 to 45 MPa in homogeneous phase and in the presence of a catalyst system containing both rhodium and aromatic phosphines in molar excess related to the rhodium, separation of the catalyst and hydrogenation of the hydroformylation product, characterised in that salts of sulfonated or carboxylated triarylphosphines which are soluble in the organic medium and insoluble in water are used as aromatic phosphines, the hydroformylation product is treated with a diluted aqueous solution of a base, the aqueous phase containing phosphine and rhodium is separated and the hydroformylation product is treated at elevated temperature with hydrogen in the presence of a hydrogenation catalyst.

2. A process according to claim 1, characterised in that the salts of sulfonated or carboxylated triarylphosphines which are soluble in organic media and insoluble in water contain $(NR_2H_2)^+$ and/or $(NR_3H)^+$ as cations, R denoting alkyl groups with 4 to 12 carbon atoms, aryl or cycloalkyl groups with 6 to 12 carbon atoms.

3. A process according to claims 1 and 2, characterised in that the hydroformylation of the oleyl alcohol takes place in the presence of 30 to 150 ppm of rhodium, related to the amount of oleyl alcohol employed, and the salts of the sulfonated or carboxylated triarylphosphines are used in a molar ratio of 5 : 1 to 200 : 1, related to the rhodium.

4. A process according to claims 1 to 3, characterised in that the hydroformylation of the oleyl alcohol takes place in the presence of 50 to 150 ppm of rhodium, related to the amount of oleyl alcohol employed, and the salts of the sulfonated or carboxylated triarylphosphines are used in a molar ratio of 40 : 1 to 100 : 1, related to the rhodium.

5. A process according to claims 1 to 4, characterised in that the catalyst system is separated with the aqueous solution of the water-soluble base at a temperature of $\leq 70°C$ and a pH value of $\geq 8$.

6. A process according to claims 1 to 5, characterised in that the catalyst system is separated with the aqueous solution of the water-soluble base at a temperature of $\leq 40°C$ and a pH value of $\geq 8.5$.

7. A process according to claims 1 to 6, characterised in that the hydroformylation product separated from the aqueous phase is hydrogenated on a nickel, cobalt or copper-containing catalyst, which may contain promoters, at temperatures of 80 to 220°C and pressures of 0.01 to 35 MPa.

8. A process according to claims 1 to 7, characterised in that the hydroformylation product separated from the aqueous phase is hydrogenated on a nickel-containing support catalyst at temperatures of 80 to 160°C and pressures of 10 to 25 MPa.

9. A process according to claims 1 to 8, characterised in that the sulfonated triarylphosphine is mono, di or trisulfonated triphenylphosphine.

10. A process according to claims 1 to 9, characterised in that the sulfonated triarylphosphine consists of a mixture of mono, di and trisulfonated triphenylphosphines.

11. A process according to claims 1 to 10, characterised in that alkali metal or alkaline earth hydroxides or an aqueous tetraalkylammonium hydroxide solution are employed as the water-soluble base.

12. A process according to claims 1 to 11, characterised in that the concentration of the base dissolved in water is 0.01 to 10 % by weight, preferably 0.5 to 5 % by weight, related to the aqueous solution.

13. A process according to claims 1 to 12, characterised in that the hydroformylation product is separated from the aqueous solution either by centrifugal force or by phase-separating apparatus with coalescing agents.

14. A process according to claims 1 to 13, characterised in that, in order to recirculate the rhodium, the aqueous phase containing the phosphine and rhodium and separated from the hydroformylation product is acidified, treated with an amine dissolved in a water-insoluble organic solvent, said amine having the formula $NHR_2$ or $NR_3$, R denoting alkyl groups with 4 to 12 carbon atoms or aryl or cycloalkyl groups with 6 to 12 carbon atoms, and after separation of the aqueous phase the organic phase is used again in the hydroformylation process.

15. A process according to claims 1 to 14, characterised in that toluene, xylene, characteristic amines $NR_2H$ or $NR_3$ or the olefin itself serve as solvents for the aromatic phosphines.

## Revendications

1. Procédé de préparation de nonadécane-diols par hydroformylation de l'alcool oléylique à des températures de 100 à 170°C et des pressions de 10 à 45 MPa en phase homogène et en présence d'un système catalyseur contenant du rhodium et des phosphines aromatiques en excès molaire par rapport au rhodium, séparation du catalyseur et hydrogénation du produit d'hydroformylation, caractérisé en ce que l'on utilise en tant que phosphines aromatiques des sels solubles dans les milieux organiques mais insolubles dans l'eau de triarylphosphines sulfonées ou carboxylées, on traite le produit d'hydroformylation par une solution aqueuse diluée d'une base, on sépare la phase aqueuse contenant la phosphine et le rhodium et on traite le produit d'hydroformylation par l'hydrogène en présence d'un catalyseur d'hydrogénation à température élevée.

2. Procédé selon la revendication 1, caractérisé en ce que l'on utilise des sels solubles dans les milieux organiques mais insolubles dans l'eau de triarylphosphines sulfonées ou carboxylées contenant en tant que cations $(NR_2H_2)^+$ et/ou $(NR_3H)^+$, R représentant des groupes alkyle en $C_4$-$C_{12}$, aryle ou cycloalkyle en $C_6$-$C_{12}$.

3. Procédé selon les revendications 1 et 2, caractérisé en ce que l'hydroformylation de l'alcool oléylique est effectuée en présence de 30 à 150 ppm de rhodium par rapport à l'alcool oléylique mis en oeuvre, et les sels de triarylphosphines sulfonées ou carboxylées sont mis en oeuvre à un rapport molaire de 5 : 1/à 200 : 1 avec le rhodium.

4. Procédé selon les revendications 1 à 3, caractérisé en ce que l'hydroformylation de l'alcool oléylique est effectuée en présence de 50 à 150 ppm de rhodium par rapport à l'alcool oléylique mis en oeuvre et en ce que les sels des triarylphosphines sulfonées ou carboxylées sont mis en oeuvre à un rapport molaire de 40 : 1 à 100 : 1 avec le rhodium.

5. Procédé selon les revendications 1 à 4, caractérisé en ce que la séparation du système catalyseur à l'aide de la solution aqueuse de la base soluble dans l'eau est réalisée à une température inférieure ou égale à 70°C et à un pH supérieur ou égal à 8.

6. Procédé selon les revendications 1 à 5, caractérisé en ce que la séparation du système catalyseur à l'aide de la solution aqueuse de la base soluble dans l'eau est effectuée à une température inférieure ou égale à 40°C et à un pH supérieur ou égal à 8,5.

7. Procédé selon les revendications 1 à 6, caractérisé en ce que le produit d'hydroformylation séparé de la phase aqueuse est hydrogéné à des températures de 80 à 220°C et des pressions de 0,01 à 35 MPa sur un catalyseur contenant du nickel, du cobalt ou du cuivre et le cas échéant des activateurs.

8. Procédé selon les revendications 1 à 7, caractérisé en ce que le produit d'hydroformylation séparé de la phase aqueuse est hydrogéné à des températures de 80 à 160°C et des pressions de 10 à 25 MPa sur un catalyseur au nickel sur support.

9. Procédé selon les revendications 1 à 8, caractérisé en ce que la triarylphosphine sulfonée consiste en triphénylphosphine mono-, di- ou tri-sulfonée.

10. Procédé selon les revendications 1 à 9, caractérisé en ce que la triarylphosphine sulfonée consiste en mélanges de triphénylphosphines mono-, di- et tri-sulfonées.

11. Procédé selon les revendications 1 à 10, caractérisé en ce que la base soluble dans l'eau qu'on utilise consiste en un hydroxyde alcalin ou alcalino-terreux ou une solution aqueuse d'hydroxyde de tétraalkylammonium.

12. Procédé selon les revendications 1 à 11, caractérisé en ce que la concentration de la base en solution dans l'eau est de 0,01 à 10 %, de préférence de 0,5 à 5 % en poids de la solution aqueuse.

13. Procédé selon les revendications 1 à 12, caractérisé en ce que le produit d'hydroformylation et la solution aqueuse sont séparés par centrifugation ou à l'aide d'appareils à séparation de phases contenant des éléments de coalescence.

14. Procédé selon les revendications 1 à 13, caractérisé en ce que, pour recycler le rhodium, on acidifie la phase aqueuse contenant la phosphine et le rhodium séparée du produit d'hydroformylation, on la traite par une amine de formule $NHR_2$ ou $NR_3$ dans laquelle R représente des groupes alkyle en $C_4$-$C_{12}$, aryle ou cycloalkyle en $C_6$-$C_{12}$, en solution dans un solvant organique insoluble dans l'eau et, après séparation de la phase aqueuse, on utilise à nouveau la phase organique à l'hydroformylation.

15. Procédé selon les revendications 1 à 14, caractérisé en ce que l'on utilise en tant que solvants pour les phosphines aromatiques le toluène, le xylène, les amines correspondantes $NR_2H$ ou $NR_3$ ou l'oléfine elle-même.